# DEMANDE DE BREVET EUROPEEN

(11) **EP 4 789 689 A1**
(43) Date de publication de la demande: **12.08.2026**
(21) Numéro de dépôt: 26157753.0
(22) Date de dépôt: 11.02.2026
(51) Int. Cl.: A61L 2/26, A61B 50/20, A61B 90/50, A61M 25/01, A61M 25/02, A61B 90/70, A61B 90/96

(54) **DISPOSITIF DE MAINTIEN D'UN INSTRUMENT MÉDICAL DANS UNE ENCEINTE DE DÉSINFECTION ET ENSEMBLE ASSOCIÉ**

(30) Priorité: 11.02.2025 FR 2501382
(71) Demandeur: Germitec, 33000 Bordeaux (FR)
(72) Inventeur: POULON, Fanny, 91440 BURES SUR YVETTE (FR); BERTHIAS, Maxime, 33750 CADARSAC (FR); DUMAS, Coralie, 07110 MONTREAL (FR)
(74) Mandataire: Lavoix

(57) **Abrégé**

La présente invention concerne un dispositif de maintien (14) d'un instrument médical dans une enceinte de désinfection, le dispositif (14) comprenant :
- deux demi-coquilles (34, 35) aptes à coopérer en une position de maintien ; et
- un dispositif de verrouillage (36) apte à maintenir les deux demi-coquilles (34, 35) dans la position de maintien autour d'une partie de l'instrument médical, le dispositif de verrouillage (36) comprenant deux éléments de verrouillage (54) complémentaires.

L'un des éléments de verrouillage comprend un premier composant d'un premier aimant, le premier composant étant d'une première polarité, l'autre des éléments de verrouillage comprenant un deuxième composant du premier aimant, le deuxième composant étant d'une deuxième polarité.

## Description

La présente invention concerne un dispositif de maintien d'un instrument médical dans un système de désinfection.

Afin d'éviter toutes contaminations, il convient de désinfecter un instrument médical entre deux utilisations.

A cet effet, il est connu de désinfecter des instruments médicaux dans des enceintes de désinfection définissant un volume de désinfection fermé. De telles enceintes comprennent par exemple un dispositif de génération de rayonnements UV (Ultra-violet) permettant notamment d'effectuer une désinfection de ces instruments médicaux, et plus particulièrement une désinfection haut-niveau.

Les instruments médicaux, tels que des sondes médicales par exemple, comprennent typiquement au moins une partie active et, généralement, également au moins une partie de raccordement sous forme de câble.

**Il** est connu de suspendre ces instruments médicaux dans l'enceinte de désinfection, par exemple à l'aide d'une bague attachée à une portion de la partie de raccordement et adaptée pour coopérer avec un support de l'enceinte de désinfection.

Une telle bague est par exemple constituée de demi-coquilles reliées entre elles par des vis.

Cependant, une telle bague n'est pas entièrement satisfaisante, notamment en raison de la présence des perçages pour les vis pouvant entrainer un défaut d'étanchéité. Un tel défaut d'étanchéité peut conduire à une hygiène insuffisante de la bague, et par exemple à une contamination par incrustation d'impuretés dans les perçages.

En outre, une telle bague est généralement compatible avec un nombre très limité de diamètres de câble de raccordement.

Par ailleurs, une telle bague a généralement une durée de vie très limitée, notamment inférieure à 10 utilisations, en particulier en raison de la durée de vie limitée des pas de vis.

En outre, il est connu d'identifier l'instrument médical dans l'enceinte de désinfection, afin d'assurer une traçabilité de la désinfection. A cet effet, l'une des demi-coquilles de la bague comprend par exemple une fenêtre derrière laquelle une étiquette d'identification, par exemple avec un code-barres, est maintenue.

Néanmoins, une telle fenêtre crée des interstices supplémentaires, ce qui renforce le risque d'une étanchéité insuffisante.

L'un des buts de l'invention est alors de pallier ces inconvénients, et notamment de proposer un dispositif étanche, durable et ergonomique permettant le maintien d'instruments médicaux variés dans une enceinte de désinfection.

A cet effet, l'invention a pour objet un dispositif de maintien d'un instrument médical dans une enceinte de désinfection, le dispositif comprenant :
- deux demi-coquilles aptes à coopérer en une position de maintien pour former ensemble un élément de maintien, chaque demi-coquille comprenant une coque s'étendant selon un axe de coque et définissant une face externe et une face interne ; et
- un dispositif de verrouillage apte à maintenir les deux demi-coquilles dans la position de maintien autour d'une partie de l'instrument médical, le dispositif de verrouillage comprenant deux éléments de verrouillage complémentaires et propre à coopérer entre eux, l'un des éléments de verrouillage étant agencé sur l'une des demi-coquilles et l'autre des éléments de verrouillage étant agencé sur l'autre des demi-coquilles.

L'un des éléments de verrouillage comprend un premier composant d'un premier aimant, le premier composant étant d'une première polarité.

L'autre des éléments de verrouillage comprend un deuxième composant du premier aimant, le deuxième composant étant d'une deuxième polarité inverse de la première polarité.

L'utilisation d'un dispositif de verrouillage avec un aimant permet de ne pas avoir à définir d'interstices supplémentaires, tels que des trous de vis par exemple, ce qui augmente l'étanchéité du dispositif.

En outre, le verrouillage par coopération de composants d'aimant est ergonomique et durable.

Suivant d'autres aspects avantageux de l'invention, le dispositif comprend une ou plusieurs des caractéristiques suivantes, prises isolément ou suivant toutes les combinaisons techniquement possibles :
- La face interne de chaque demi-coquille définit un logement,
   dans la position de maintien, les deux logements formant ensemble un espace de réception de la partie de l'instrument médical.
- Le dispositif comprend en outre un joint configuré pour être arrangé entre la partie de l'instrument médical et l'élément de maintien.
- Le joint est configuré pour s'étendre dans l'intégralité de l'espace de réception formé par les deux logements en position de maintien.
- L'un des éléments de verrouillage comprend également un premier composant d'un deuxième aimant, le premier composant étant d'une première polarité, l'autre des éléments de verrouillage comprenant également un deuxième composant du deuxième aimant, le deuxième composant étant d'une deuxième polarité.
- Le premier aimant et/ou le deuxième aimant sont des super-aimants en néodyme.
- La face interne de chaque demi-coquille définit au moins une cavité remplie par un agent de remplissage.
- Chaque élément du premier aimant, et le cas échéant chaque élément du deuxième aimant, est arrangée dans la ou l'une des cavités de la demi-coquille correspondante.
- Le dispositif comprend un élément d'identification gravé sur la face externe de l'une des demi-coquilles.

L'invention concerne également un ensemble comprenant :
- un dispositif tel que décrit ci-dessus, et
- un instrument médical comprenant une partie apte à être maintenue dans l'élément de maintien formé par les deux demi-coquilles.

L'invention apparaîtra plus clairement à la lecture de la description qui va suivre, donnée uniquement à titre d'exemple non limitatif, et faite en référence aux dessins dans lesquels :
La Figure 1 est une représentation schématique d'un ensemble selon l'invention dans une enceinte de désinfection, l'ensemble comprenant un dispositif de maintien,
La Figure 2 est une représentation schématique en éclaté du dispositif de maintien de la Figure 1, et
La Figure 3 est une représentation schématique d'une des demi-coquilles du dispositif de maintien de la Figure 1.

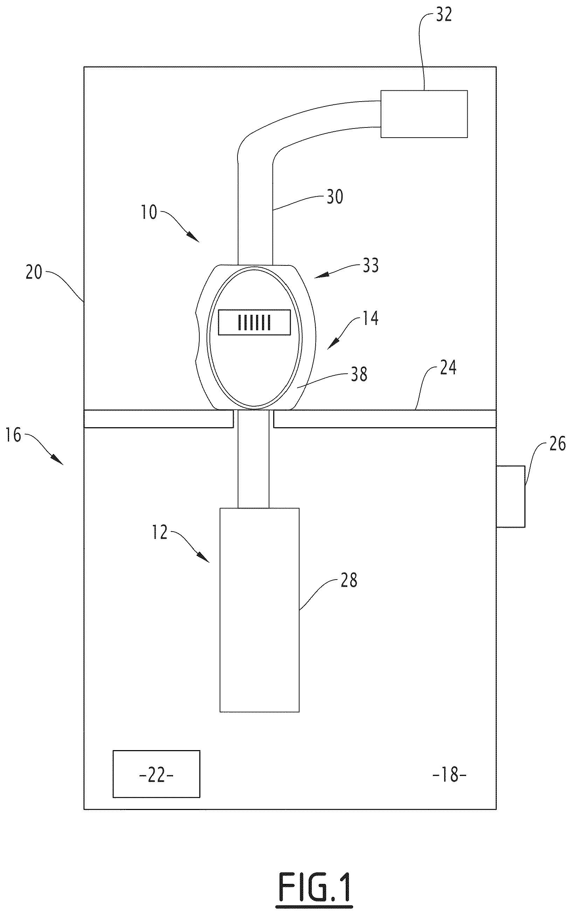

La Figure 1 montre un ensemble 10 comprenant un instrument médical 12 et un dispositif de maintien 14 dans une enceinte de désinfection 16.

En particulier, sur la Figure 1, l'instrument médical 12 est maintenu dans l'enceinte de désinfection 16 par le dispositif de maintien 14.

Par exemple, l'enceinte de désinfection 16 comprend un volume de désinfection 18 délimité par au moins une paroi 20, une unité de désinfection 22 et au moins un support 24.

De préférence, l'enceinte de désinfection 16 comprend en outre une unité d'identification 26.

L'unité de désinfection 22 est par un exemple un dispositif de génération de rayonnements de génération de rayonnements UV, de préférence UV-C.

Plus précisément, l'unité de désinfection 22 est par exemple configurée pour générer des rayonnements UV, et notamment UV-C, dans le volume de désinfection 18.

Le support 24 est, de préférence, configuré pour coopérer avec le dispositif de maintien 14 de sorte à maintenir l'instrument médical 12 à l'intérieur du volume de désinfection 18.

L'unité d'identification 26 est par exemple configurée de sorte à identifier l'instrument médical 12.

Par « identifier », on entend plus particulièrement la lecture d'informations d'identification de l'instrument médical 12.

Dans un mode de réalisation particulier, l'unité d'identification 26 est un lecteur de code-barres, par exemple agencée sur une face externe de l'une des parois de l'enceinte de désinfection 16.

L'instrument médical 12 est par exemple une sonde médicale, notamment une sonde médicale échographique, et plus particulièrement une sonde médicale échographique par voie endocavitaire, telle que par exemple une sonde gynécologique.

L'instrument médical 12 comprend par exemple une partie active 28. La partie active 28 est par exemple un tube destiné à être inséré dans le patient.

L'instrument médical 12 comprend, en outre, de préférence une partie de raccordement 30 et de manière encore plus préférentielle également un connecteur 32.

La partie de raccordement 30 est par exemple un câble de raccordement reliant la partie active 28 au connecteur 32, comme cela est notamment visible sur la Figure 1.

Plus particulièrement, la partie de raccordement 30 est par exemple un câble de diamètre compris entre 3 mm et 10 mm.

Le connecteur 32 est par exemple configuré pour être connecté à une unité de mesure et/ou d'affichage, de sorte notamment à collecter et/ou afficher des informations captées par la partie active 28.

Le dispositif de maintien 14 comprend deux demi-coquilles 34, 35 et un dispositif de verrouillage 36.

Comme cela est visible sur la Figure 1, les deux demi-coquilles 34, 35 sont aptes à coopérer en une position de maintien, en particulier pour former ensemble un élément de maintien 33 (visible sur la Figure 1), par exemple autour d'une partie de l'instrument médical 12, et plus particulièrement autour de la partie de raccordement 30 de l'instrument médical 12.

L'élément de maintien 33 ainsi formé a par exemple une section transversale de forme sensiblement circulaire ou ovale.

Le dispositif de verrouillage 36 est apte à maintenir les deux demi-coquilles 34, 35 dans la position de maintien autour d'une partie de l'instrument médical 12, et en particulier à maintenir l'élément de maintien 33 autour de la partie de l'instrument médical 12.

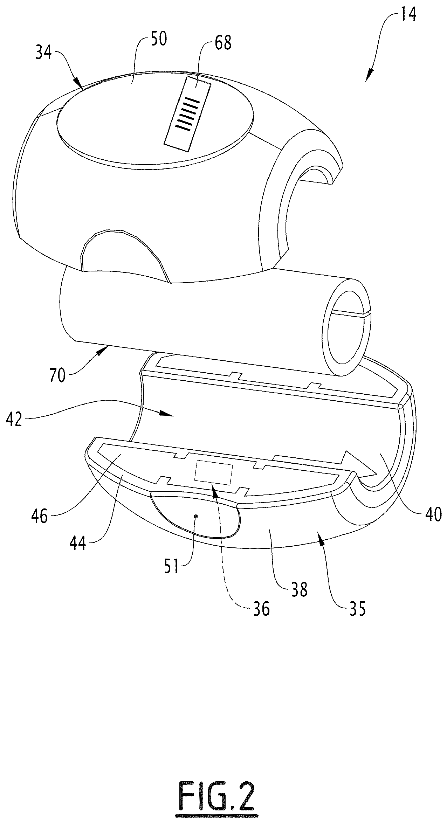

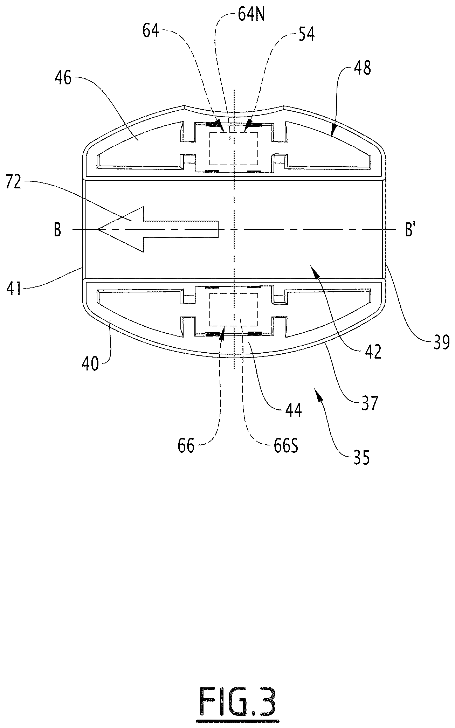

La Figure 3 montre l'une des demi-coquilles 35 du dispositif de maintien 14 de la Figure 2.

Les deux demi-coquilles 34, 35 du dispositif de maintien 14 sont, de préférence, sensiblement identiques.

Chaque demi-coquille 34, 35 comprend une coque 37 s'étendant selon un axe de coque B-B'.

Chaque coque 37 est par exemple fabriquée en plastique.

Lorsque les demi-coquilles 34, 35 coopèrent ensemble pour former l'élément de maintien 33 autour d'une partie de l'instrument médical 12, l'axe de coque B-B' est de préférence confondu avec l'axe de la partie de raccordement 30 de l'instrument médical 12.

Chaque demi-coquille 34, 35 définit une première extrémité 39 et une deuxième extrémité 41 opposée à la première extrémité 39 selon l'axe de coque B-B'.

Comme illustré sur les figure 1, la première extrémité 39 et la deuxième extrémité 41 correspondent par exemple à une extrémité haute et une extrémité basse de la coque 37, lorsque les demi-coquilles 34, 35 coopèrent pour former l'élément de maintien 33 autour de la partie de raccordement 30 de l'instrument médical 12 s'étendant verticalement.

Chaque demi-coquille 34, 35 définit une face externe 38 (visible sur les Figures 1 et 2) dirigée radialement vers l'extérieur et une face interne 40 (visible sur les Figures 2 et 3) dirigée radialement vers l'intérieur, et plus particulièrement destinée à être dirigée vers la partie de raccordement 30 de l'instrument médical 12.

Plus précisément, une partie de la face interne 40 est destinée à venir contre l'instrument médical 12, et en particulier contre la partie de raccordement 30 de l'instrument médical 12, ou à venir contre le joint 70 (qui sera décrit ultérieurement).

Les faces internes 40 de chaque demi-coquille 34, 35 sont avantageusement aptes à venir partiellement en contact l'une contre l'autre lors de la formation de l'élément de maintien 33.

En particulier, chacune des faces internes 40 comprend une partie destinée à venir contre l'instrument médical 12 ou contre le joint 70 et avantageusement au moins une autre partie destinée à venir contre la face interne 40 de l'autre demi-coquille 34, 35.

De préférence, la face interne 40 de chaque demi-coquille 34, 35 définit un logement 42.

Comme illustré sur les figures 2 et 3, le logement 42 s'étend de préférence selon l'axe de coque B-B' de la première extrémité 39 à la deuxième extrémité 41.

Le logement 42 a par exemple une longueur, notamment correspondant à la distance entre la première extrémité 39 et la deuxième extrémité 41, comprise entre 30 mm et 60 mm.

Par exemple, le logement 42 présente une section en forme de demi-cercle de diamètre légèrement supérieur à de la partie de raccordement 30 de l'instrument médical 12, notamment compris entre 10 mm et 15 mm.

De préférence, dans la position de maintien, les deux logements 42 forment ensemble un espace de réception de la partie de l'instrument médical 12, l'espace de réception étant par exemple cylindrique.

Comme cela est visible sur les Figures 2 et 3, la face interne 40 de chaque demi-coquille 34, 35 définit avantageusement deux rebords 44 s'étendant de chaque côté du logement 42.

De préférence, les rebords 44 des faces internes 40 de chaque demi-coquille 34, 35 viennent en contact l'un avec l'autre lors de la formation de l'élément de maintien 33.

Avantageusement, comme cela est visible sur les figures 2 et 3, la face interne 40 de chaque demi-coquille 34, 35 définit au moins une cavité 46, et de préférence deux cavités 46.

De manière préférentielles, chaque cavité 46 est ménagée dans l'un des rebords 44 de la face interne 40 de la coque 37.

Avantageusement, chaque cavité 46 est remplie par un agent de remplissage 48, et de préférence complètement remplie.

Par complétement remplie, on entend que de l'agent de remplissage 48 est présent dans le volume total de la cavité 46.

En particulier, la cavité 46 est remplie de sorte que l'agent de remplissage 48 affleure le haut du rebord 44.

L'agent de remplissage 48 est par exemple de la résine, notamment de la résine époxy.

La face externe 38 de chaque demi-coquille 34, 35 comprend par exemple un méplat 50, par exemple de forme circulaire ou ovale.

De préférence, la face externe 38 de chaque demi-coquille 34, 35 comprend un évidement 51, de préférence de forme de demi-cercle.

Avantageusement en position de maintien, les deux évidement 51 forment ensemble une échancrure de forme sensiblement circulaire, adaptée pour le positionnement d'un pouce de l'utilisateur.

Le dispositif de verrouillage 36 comprend deux éléments de verrouillage 54 complémentaires et propre à coopérer entre eux, l'un des éléments de verrouillage étant agencé sur l'une des demi-coquilles 34 et l'autre des éléments de verrouillage 54 étant agencé sur l'autre des demi-coquilles 35.

Par coopération des éléments de verrouillage 54 du dispositif de verrouillage 36, l'élément de maintien 33 formé par les deux demi-coquilles 34, 35 est apte à être maintenu sur la partie de l'instrument médical 12 et plus particulièrement comme expliqué précédemment selon la direction radiale.

L'un des éléments de verrouillage comprend un premier composant d'un premier aimant 64, le premier composant étant d'une première polarité, correspondant par exemple au pôle sud magnétique.

L'autre des éléments de verrouillage 54 comprend un deuxième composant 64N du premier aimant 64, le deuxième composant 64N étant d'une deuxième polarité inverse de la première polarité, correspondant par exemple au pôle nord magnétique.

Dans un mode de réalisation préféré visible sur les Figures 1 et 2, l'un des éléments de verrouillage 54 comprend également un premier composant d'un deuxième aimant 66, le premier composant 66S étant d'une première polarité, correspondant par exemple au pôle sud magnétique. L'autre des éléments de verrouillage comprend également un deuxième composant du deuxième aimant 66, le deuxième composant étant d'une deuxième polarité, correspondant par exemple au pôle nord magnétique.

De préférence, le premier aimant 64 et/ou le deuxième aimant 66 sont des super-aimants en néodyme.

Chacun du premier composant et du deuxième composant 64N du premier aimant 64, et respectivement chacun du premier composant 66S et du deuxième composant du deuxième aimant 66 le cas échéant, sont plus particulièrement agencés en regard l'un de l'autre dans la position de maintien, notamment de sorte à créer une force d'attraction magnétique entre eux fixant les deux demi-coquilles 34, 35 entre elles.

Chacun du premier et du deuxième 64N composants du premier aimant 64, et respectivement chacun du premier 66S et du deuxième composant du deuxième aimant 66 le cas échéant, est par exemple agencé sur l'un des rebords 44 de la face interne 40 de la demi-coquille 34, 35 correspondante.

Plus précisément, chaque composant 64N du premier aimant 64, et de préférence chaque composant 66S du deuxième aimant 66, est arrangée dans l'une des cavités 46 ménagées dans la face interne 40 de la coque 37 correspondante.

De préférence, chaque composant 64N du premier aimant 64, et de préférence chaque composant 66S du deuxième aimant 66, est complétement noyé dans l'agent de remplissage 48 dans la cavité 46 correspondante.

Par complétement noyé, on entend que l'agent de remplissage 48 recouvre entièrement le composant dans la cavité 46.

De manière préférentielle, le dispositif de maintien 14 comprend, en outre, un élément d'identification 68.

En particulier, l'élément d'identification 68 comprend des informations d'identification, dont par exemple un numéro d'identification du dispositif de maintien 14 et/ou de l'instrument médical 12 destiné à être désinfecter.

L'élément d'identification 68 est par exemple un code-barres.

De préférence, l'élément d'identification 68 est gravé sur la face externe 38 de l'une des demi-coquilles 34, 35, et par exemple par gravure laser.

Par exemple, comme cela est visible sur les Figures 1 et 2, l'élément d'identification 68 est gravé sur le méplat 50 de de l'une des demi-coquilles 34, 35.

De manière préférentielle, le dispositif de maintien 14 comprend, en outre, un joint 70 configuré pour être arrangé entre la partie de l'instrument médical 12 et l'élément de maintien 33.

En particulier, le joint 70 est configuré pour enserrer la portion de la partie de raccordement 30 destinée à être reçu dans l'espace de réception formé par les deux logements 42 dans la position de maintien.

Avantageusement, le joint 70 est configuré pour s'étendre dans l'intégralité dudit espace de réception formé par les deux logements 42 en position de maintien, ce qui permet notamment de réduire le risque de pénétration de liquide dans l'élément de maintien.

De préférence, le joint 70 a une longueur sensiblement identique à celles des logements 42.

Par exemple, comme cela est visible sur la Figure 2, le joint 70 est un cylindre fendu, en particulier apte à venir s'insérer autour de la partie de raccordement 30 de l'instrument médical 12.

De manière avantageuse, le joint 70 présente une section annulaire avec un diamètre interne sensiblement égal à celui de la partie de raccordement 30 et un diamètre externe sensiblement égal à celui des logements 42.

Le joint 70 est par exemple fabriqué en un matériau comprenant de la silicone, et en particulier en un caoutchouc de silicone.

De manière avantageuse, le dispositif de maintien 14 comprend également au moins un pictogramme 72, par exemple en forme de flèche comme cela est visible sur la Figure 2.

Ce pictogramme 72 est de préférence gravé, par exemple par gravure laser, sur l'une des faces 38, 40 de l'une des demi-coquilles 34, 35.

Par exemple, le pictogramme 72 est gravé sur la face interne 40 l'une des demi-coquilles 34, 35, et en particulier dans le logement 42 correspondant, de sorte à indiquer à l'utilisateur un sens d'insertion de la partie de raccordement 30.

Un exemple d'utilisation d'un tel dispositif de maintien 14 va maintenant être décrit.

Un dispositif de maintien 14 tel que décrit ci-dessus et un instrument médical 12 destiné à être désinfecté sont tout d'abord fournis.

De manière préférentielle, le joint 70 est fixé sur l'instrument médical 12.

Plus particulièrement, le joint 70 est fixé autour d'une portion de la partie de raccordement 30 de l'instrument médical 12.

Une partie de l'instrument médical 12, en particulier l'ensemble formée par le joint 70 et la portion de la partie de raccordement 30 entourée par celui-ci, est positionné(e) dans le logement 42 de l'une des demi-coquilles 34, 35.

L'autre des demi-coquilles 35 est ensuite fixée à celle-ci en position de maintien par le dispositif de verrouillage 36, de sorte à former l'élément de maintien 33.

Par exemple, le dispositif de maintien 14 est identifié, notamment par lecture des informations sur l'élément d'identification 68 par l'unité d'identification 26 de l'enceinte de désinfection 16.

L'ensemble formé par le dispositif de maintien 14 et l'instrument médical 12 est ensuite agencé dans l'enceinte de désinfection 16, notamment par coopération entre l'élément de maintien 33 et le support 24 comme cela est visible sur la Figure 1, de sorte à suspendre l'instrument médical 12.

Un cycle de désinfection de l'instrument médical 12 est ensuite par exemple mis en œuvre, notamment par l'unité de désinfection 22.

Un tel dispositif de maintien 14 est particulièrement avantageux.

En particulier, l'utilisation d'un dispositif de verrouillage 36 avec au moins un aimant 64, 66 permet de ne pas avoir à définir d'interstices supplémentaires, tels que des trous de vis par exemple, ce qui augmente l'étanchéité du dispositif.

De même, la suppression de la fenêtre pour l'identification en gravant l'élément d'identification 68 renforce également cette étanchéité.

Par ailleurs, la conception des demi-coquilles 34, 35 sans creux, notamment en noyant les composants 64N, 66S des aimants 64, 66 dans l'agent de remplissage 48, permet encore plus de renforcer cette étanchéité.

Un tel dispositif de maintien 14 présente donc une étanchéité optimisée, ce qui permet de réduire les risques de contamination.

Par ailleurs, l'absence d'interstices et de creux facilite le nettoyage du dispositif de maintien 14.

En outre, le verrouillage par coopération de composants 64N, 66S d'aimants 64, 66 est simple à mettre en œuvre et est ergonomique.

L'ajout optionnelle des évidements 51 sur les demi-coquilles 34, 35 facilite encore plus la manipulation d'un tel dispositif de maintien 14 et le rend encore plus ergonomique.

Par ailleurs, par la suppression du pas de vis grâce à l'utilisation d'aimants 64, 66, la durabilité d'un tel dispositif de maintien est fortement augmenté.

En outre, la gravure de l'élément d'identification 68 renforce son intégrité physique, et évite ainsi que celui-ci devienne illisible, ce qui limite les risques de mauvaise identification.

L'utilisation du joint 70 permet en outre de rendre ce dispositif de maintien 14 compatible avec un grand nombre de diamètres de partie de raccordement 30, notamment en choisissant une épaisseur de joint 70 adaptée.

En outre, un tel dispositif de maintien 14 est simple à fabriquer et économique, notamment en raison de son faible nombre de composants.

L'homme du métier comprendra que les modes de réalisation et variantes précédemment décrits peuvent être combinés entre eux pourvu qu'ils soient compatibles techniquement.

## Revendications

1. Dispositif de maintien (14) d'un instrument médical (12) dans une enceinte de désinfection (16), le dispositif (14) comprenant :
- deux demi-coquilles (34, 35) aptes à coopérer en une position de maintien pour former ensemble un élément de maintien (33), chaque demi-coquille (34, 35) comprenant une coque (37) s'étendant selon un axe de coque (B-B') et définissant une face externe (38) et une face interne (40) ; et
- un dispositif de verrouillage (36) apte à maintenir les deux demi-coquilles (34, 35) dans la position de maintien autour d'une partie de l'instrument médical (12), le dispositif de verrouillage (36) comprenant deux éléments de verrouillage (54) complémentaires et propre à coopérer entre eux, l'un des éléments de verrouillage étant agencé sur l'une des demi-coquilles (34) et l'autre des éléments de verrouillage (54) étant agencé sur l'autre des demi-coquilles (35) ;
**caractérisé en ce que**
l'un des éléments de verrouillage comprend un premier composant d'un premier aimant (64), le premier composant étant d'une première polarité,
l'autre des éléments de verrouillage (54) comprenant un deuxième composant (64N) du premier aimant (64), le deuxième composant (64N) étant d'une deuxième polarité inverse de la première polarité.

2. Dispositif selon la revendication 1, dans lequel la face interne (40) de chaque demi-coquille (34, 35) définit un logement (42),
dans la position de maintien, les deux logements (42) formant ensemble un espace de réception de la partie (30) de l'instrument médical (12).

3. Dispositif selon la revendication 1 ou 2, comprenant en outre un joint (70) configuré pour être arrangé entre la partie de l'instrument médical (12) et l'élément de maintien (33).

4. Dispositif selon les revendications 2 et 3, dans lequel le joint (70) est configuré pour s'étendre dans l'intégralité de l'espace de réception formé par les deux logements (42) en position de maintien.

5. Dispositif selon l'une quelconque des revendications 1 à 4, dans lequel l'un des éléments de verrouillage (54) comprend également un premier composant (66S) d'un deuxième aimant (66), le premier composant (66S) étant d'une première polarité, l'autre des éléments de verrouillage comprenant également un deuxième composant du deuxième aimant (66), le deuxième composant étant d'une deuxième polarité.

6. Dispositif selon l'une quelconque des revendications 1 à 5, dans lequel le premier aimant (64) et/ou le deuxième aimant (66) sont des super-aimants en néodyme.

7. Dispositif selon l'une quelconque des revendications 1 à 6, dans lequel la face interne (40) de chaque demi-coquille (34, 35) définit au moins une cavité (46) remplie par un agent de remplissage (48).

8. Dispositif selon la revendication 7, dans lequel chaque élément (64N) du premier aimant (64), et le cas échéant chaque élément (66S) du deuxième aimant (66), est arrangée dans la ou l'une des cavités (46) de la demi-coquille (34, 35) correspondante.

9. Dispositif selon l'une quelconque des revendications 1 à 8, comprenant un élément d'identification (68) gravé sur la face externe (38) de l'une des demi-coquilles (34, 35).

10. Ensemble (10) comprenant :
- un dispositif (14) selon l'un quelconque des revendications précédentes, et
- un instrument médical (12) comprenant une partie (30) apte à être maintenue dans l'élément de maintien (33) formé par les deux demi-coquilles (34, 35).
